(19) **Europäisches Patentamt / European Patent Office / Office européen des brevets**

(11) **EP 4 302 873 A1**

(12) # EUROPEAN PATENT APPLICATION
published in accordance with Art. 153(4) EPC

(43) Date of publication:
**10.01.2024 Bulletin 2024/02**

(21) Application number: **22763088.6**

(22) Date of filing: **24.02.2022**

(51) International Patent Classification (IPC):
**B01J 23/887** (2006.01)       **B01J 37/04** (2006.01)
**C07B 61/00** (2006.01)        **C07C 45/35** (2006.01)
**C07C 47/22** (2006.01)        **C07C 51/25** (2006.01)
**C07C 57/05** (2006.01)

(52) Cooperative Patent Classification (CPC):
**B01J 23/887; B01J 37/04; C07B 61/00;
C07C 45/35; C07C 47/22; C07C 51/25; C07C 57/04**

(86) International application number:
**PCT/JP2022/007535**

(87) International publication number:
**WO 2022/186032 (09.09.2022 Gazette 2022/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.03.2021   JP 2021033249**

(71) Applicant: **Nippon Kayaku Kabushiki Kaisha
Tokyo 100-0005 (JP)**

(72) Inventors:
• **OKUMURA, Shigeki
  Sanyoonoda-shi, Yamaguchi 757-8686 (JP)**

• **YASUDA, Shogo
  Sanyoonoda-shi, Yamaguchi 757-8686 (JP)**
• **NAKANO, Takanori
  Sanyoonoda-shi, Yamaguchi 757-8686 (JP)**
• **HINO, Yumi
  Sanyoonoda-shi, Yamaguchi 757-8686 (JP)**
• **KAWAMURA, Tomoyuki
  Tokyo 100-0005 (JP)**

(74) Representative: **Gille Hrabal
Partnerschaftsgesellschaft mbB
Patentanwälte
Brucknerstraße 20
40593 Düsseldorf (DE)**

(54) **CATALYST, AND METHOD FOR PRODUCING COMPOUND THROUGH GAS-PHASE OXIDATION REACTION USING SAME**

(57)     A catalyst containing molybdenum, bismuth, and iron, in which R1 represented by the following equation (1) is 0.45 or more and 5.00 or less,

$$R1 = (\text{maximum value of peak at } 886 \text{ cm}^{-1} \pm 5 \text{ cm}^{-1}) \div (\text{maximum value of peak at } 354 \text{ cm}^{-1} \pm 5 \text{ cm}^{-1})$$

as measured by Raman spectroscopy          (1).

EP 4 302 873 A1

**FIG. 1**

WAVE NUMBER /cm⁻¹

**Description**

TECHNICAL FIELD

The present invention relates to a catalyst and a gas phase oxidation reaction using the catalyst, particularly a method for producing an unsaturated aldehyde and/or an unsaturated carboxylic acid or a conjugated diene.

BACKGROUND ART

A method for producing a corresponding unsaturated aldehyde or unsaturated carboxylic acid using propylene, isobutylene, t-butyl alcohol, or the like as a raw material, or a catalytic gas phase oxidation method for producing 1,3-butadiene from butenes is widely practiced industrially. In particular, regarding the method for producing a corresponding acrolein or acrylic acid using propylene as a raw material, many reports have been made as means for improving the yield (for example, Patent Literatures 1 and 2).

Patent Literature 3 discloses that when two or more types of composite metal oxide catalysts having different compositions are prepared and two or more layers are stacked in a tube axis direction for multi-layer filling, the catalyst is filled such that the amount of a bismuth component with respect to molybdenum decreases from a gas inlet side toward a gas outlet side, and the amount of an iron component with respect to molybdenum increases from the gas inlet side toward the gas outlet side, whereby a salt bath temperature (reaction temperature) is kept low even in a reaction under high load conditions. In the related art, in a step of producing a corresponding unsaturated aldehyde and/or unsaturated carboxylic acid from alkenes, there is no study on improvement in a total yield (hereinafter referred to as an "useful yield") of the unsaturated aldehyde and/or unsaturated carboxylic acid, and on stable production while maintaining an improved useful yield while keeping the salt bath temperature low even in a reaction under high load conditions.

In addition, Patent Literature 4 proposes a method of controlling a peak ratio measured by Raman spectroscopy to a specific numerical range. However, in Patent Literature 4, a method for producing a catalyst is complicated, the catalyst performance required as a practical catalyst is insufficient, and further improvement in catalyst performance and elucidation of physical property data which may be a cause thereof have been waited for.

CITATION LIST

PATENT LITERATURE

Patent Literature 1: WO 2016/136882
Patent Literature 2: JP2017-024009A
Patent Literature 3: WO 2015/008815
Patent Literature 4: JP2020-142221A

SUMMARY OF INVENTION

TECHNICAL PROBLEM

Even when improvement is made by means as described above, further improvement in yield is required in the production of a corresponding unsaturated aldehyde and/or unsaturated carboxylic acid by a partial oxidation reaction of alkenes such as propylene and isobutylene, and t-butyl alcohol. For example, the yield of a target product influences an amount of alkenes such as propylene and isobutylene, and t-butyl alcohol for use in the production and greatly influences the production cost. In addition, continuing the operation at a low yield causes a large amount of by-products and this applies a large load in a refining step. Thus, it may increase the time and the operating cost required for the refining step. Further, some types of the by-products may be deposited on the surface of the catalyst or in a gas flow path near the catalyst. This reduces the activity of the catalyst by covering necessary reaction active sites on the surface of the catalyst, and thus this results in increasing the salt bath temperature for forcibly increasing the activity. Then, the catalyst is subjected to thermal stress, and the thermal stress causes a decrease in lifetime and a further decrease in selectivity. This results in a decrease in yield. Further, the by-products deposited in the system may also cause an increase in internal pressure, and the increase may also lead to a decrease in selectivity and a decrease in yield. In some cases, a sudden increase in internal pressure causes a temperature abnormality and the reaction runs out of control. This case may cause the operation to be stopped for a long period of time and it may become necessary to clean the inside of the system and replace the catalyst.

SOLUTION TO PROBLEM

Therefore, an object of the present invention is to provide a catalyst with which an unsaturated aldehyde, an unsaturated carboxylic acid, or a conjugated diene can be produced with a high selectivity and a high yield.

That is, the present invention relates to the following 1) to 8).

1) A catalyst containing molybdenum, bismuth, and iron, in which R1 represented by the following equation (1) is 0.45 or more and 5.00 or less.
[Math. 1]

$$R1 = (\text{maximum value of peak at } 886\,\text{cm}^{-1} \pm 5\,\text{cm}^{-1}) \div (\text{maximum value of peak at } 354\,\text{cm}^{-1} \pm 5\,\text{cm}^{-1}) \text{ as measured by Raman spectroscopy} \qquad (1)$$

2) A catalyst containing molybdenum, bismuth, and iron, in which R2 represented by the following equation (2) is 951.4 cm$^{-1}$ or more and 959.0 cm$^{-1}$ or less.
[Math. 2]

$$R2 = \text{wave number (cm}^{-1})\text{ for giving maximum value of maximum peak at } 952\,\text{cm}^{-1} \pm 7\,\text{cm}^{-1} \text{ as measured by Raman spectroscopy} \qquad (2)$$

3) A catalyst containing molybdenum, bismuth, and iron, in which R3 represented by the following equation (3) is 0.21 or more.
[Math. 3]

$$R1 = (\text{maximum value of peak at } 886\,\text{cm}^{-1} \pm 5\,\text{cm}^{-1}) \div (\text{maximum value of peak at } 354\,\text{cm}^{-1} \pm 5\,\text{cm}^{-1}) \text{ as measured by Raman spectroscopy} \qquad (1)$$

[Math. 4]

$$R2 = \text{wave number (cm}^{-1})\text{ for giving maximum value of maximum peak at } 952\,\text{cm}^{-1} \pm 7\,\text{cm}^{-1} \text{ as measured by Raman spectroscopy} \qquad (2)$$

[Math. 5]

$$R3 = 2.95 \times R1 + 0.33 \times R2 - 315 \qquad (3)$$

4) The catalyst according to any one of the above 1) to 3), in which a composition of a catalytically active component is represented by the following formula (I):
[Chem. 1]

$$Mo_{a1}Bi_{b1}Ni_{c1}Co_{d1}Fe_{e1}X_{f1}Y_{g1}Z_{h1}O_{i1} \qquad (I)$$

(in the formula, Mo, Bi, Ni, Co, and Fe respectively represent molybdenum, bismuth, nickel, cobalt, and iron; X is at least one element selected from tungsten, antimony, tin, zinc, chromium, manganese, magnesium, silicon, aluminum, cerium, and titanium; Y is at least one element selected from sodium, potassium, cesium, rubidium, and thallium; Z belongs to Groups 1 to 16 in the periodic table, and means at least one element selected from elements other than the above Mo, Bi, Ni, Co, Fe, X, and Y; a1, b1, c1, d1, e1, f1, g1, h1, and i1 respectively represent the number of atoms of molybdenum, bismuth, nickel, cobalt, iron, X, Y, Z, and oxygen; and when a1 = 12, $0 < b1 \le 7$, $0 \le c1 \le 10$, $0 < d1 \le 10$, $0 < c1+d1 \le 20$, $0 \le e1 \le 5$, $0 \le f1 \le 2$, $0 \le g1 \le 3$, $0 \le h1 \le 5$, and i1 is a value determined by an oxidation state of each element).

5) The catalyst according to any one of the above 1) to 4), in which the catalytically active component is carried on an inert carrier.

6) The catalyst according to the above 5), in which the inert carrier is silica, alumina, or a combination thereof.

7) A method for producing the catalyst according to any one of the above 1) to 6), including:
a blending step of adding each of a source compound containing molybdenum, a source compound containing bismuth and a source compound containing iron to a solvent or solution and performing integration and heating to form a blended solution, in which, in the blending step, pH of the blended solution is adjusted to a range of 1.0 to 7.5 before adding an iron raw material.
8) A method for producing an unsaturated aldehyde and/or an unsaturated carboxylic acid using the catalyst according to any one of the above 1) to 6).

ADVANTAGEOUS EFFECTS OF INVENTION

The catalyst according to the present invention maintains a high selectivity and is very effective in improving the yield in a catalytic gas phase oxidation reaction or catalytic gas phase oxidative dehydrogenation reaction. The catalyst according to the present invention is particularly useful in production of a corresponding unsaturated aldehyde and unsaturated carboxylic acid using propylene, isobutylene, t-butyl alcohol, or the like as a raw material.

BRIEF DESCRIPTION OF DRAWINGS

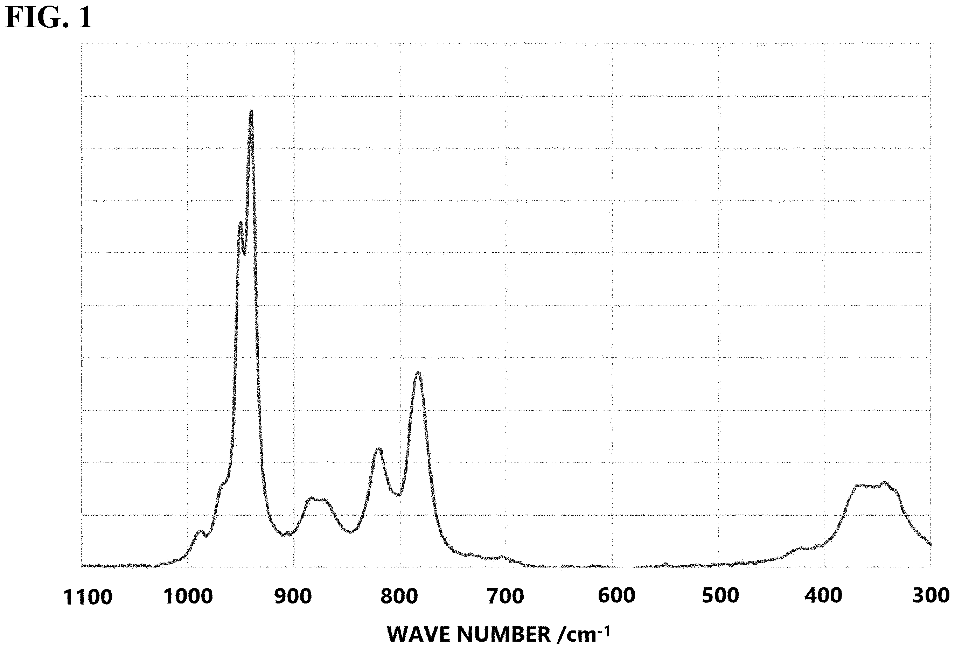

FIG. 1 is a diagram showing results of Raman spectroscopic measurement for catalyst 1 in Example 1.

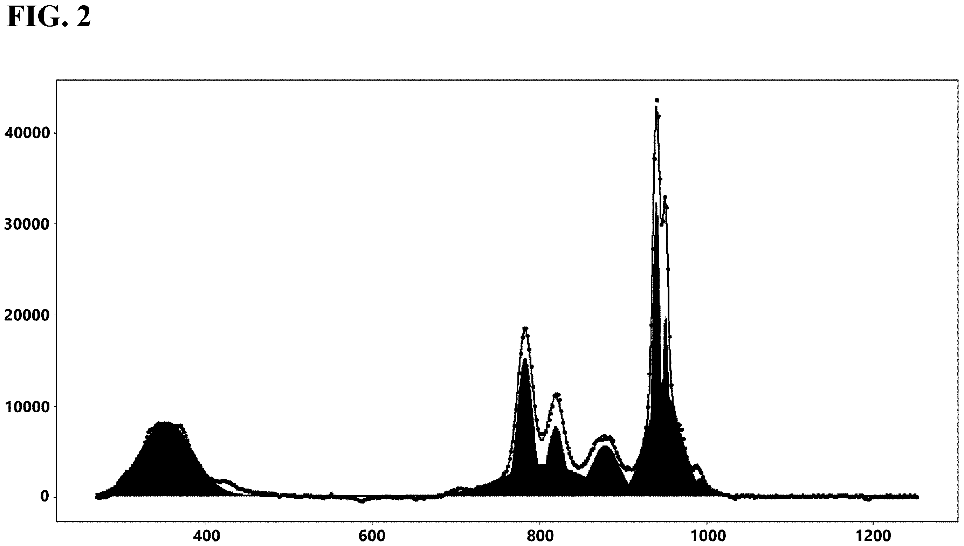

FIG. 2 is a diagram showing, as an example, a result of performing peak separation on FIG. 1 using a Gaussian function. In the figure, the points are actual data, the filled regions are fitted Gaussian functions, and the solid line is a fitting curve obtained by adding the points and the filled regions.

DESCRIPTION OF EMBODIMENTS

Hereinafter, the present invention will be described in detail. In the present description, the catalytic gas phase oxidation reaction and the catalytic gas phase oxidative dehydrogenation reaction may be collectively referred to simply as an oxidation reaction.

[R1]

A catalyst according to the present invention is characterized in that R1 represented by the above equation (1) is 0.45 or more and 5.00 or less.
R1 is a value obtained by dividing a maximum value of a peak at $886$ $cm^{-1}$ $\pm$ $5$ $cm^{-1}$ by a maximum value of a peak at $354$ $cm^{-1}$ $\pm$ $5$ $cm^{-1}$ measured by Raman spectroscopy, and the essence lies in the maximum value of the peak at $886$ $cm^{-1}$ $\pm$ $5$ $cm^{-1}$. Although this peak is a peak not assigned in the literature and the details are unknown, the present inventors consider that it is associated with the amount of an O-Mo bond in a molybdate ($MoO_4$). The present inventors have found that high R1 indicates low activity and a high selectivity, and low R1 indicates high activity and a low selectivity, so that there is an optimal numerical range for R1.
That is, the catalyst according to the present invention is based on the finding that a high selectivity and a high yield can be achieved when R1 is 0.45 or more and 5.00 or less.
The lower limit of R1 is more preferably 0.46, and is 0.47, 0.48, 0.49, 0.50, 0.51, and 0.52 in order of preference. The upper limit of R1 is more preferably 4.50, and is 4.25, 4.00, 3.75, 3.50, 3.25, 3.00, 2.75, 2.50, 2.25, 2.00, 1.75, 1.50, 1.25, 1.00, 0.75, 0.70, 0.65, 0.60, and 0.55 in order of preference. That is, a more preferred range for the value of R1 is set by the above upper and lower limits, and is, for example, 0.51 or more and 3.00 or less, and most preferably 0.52 or more and 0.55 or less.
The measurement by Raman spectroscopy can be performed by a known method, for example, using the following equipment. WP 785 Raman Spectrometer manufactured by Wasatch Photonics is used as a Raman spectrometer, I0785MM0350MF manufactured by Innovative Photonic Solutions is used as a laser module, WP785-RP manufactured by Wasatch Photonics is used as a non-contact probe, Panorama Wasatch 4.0 manufactured by Wasatch Photonics is used as software for analysis, and the Raman spectrum measurement is performed under the conditions of an excitation laser wavelength of 785 nm, a laser power of 240 mW to 480 mW, a laser irradiation time of 50 ms, and the number of times of integration of 64 times. The catalyst to be measured is directly exposed to the probe without a special treatment, but when the surrounding noise is large, the measurement is performed in a dark room. The data obtained by the measurement are subjected to background correction using the software described above, and fitted with a Gaussian function (equation (4)) to separate peaks.
[Math. 6]

$$y = (i'-*) \times \exp\{-(x-(p-*))^2 \div (w-*)^2\} \qquad (4)$$

y: Gaussian function (function for wave number $x/cm^{-1}$)
p-*: peak position of maximum value for giving *-th peak/$cm^{-1}$
w-*: peak width at *-th peak/$cm^{-1}$
i'-*: (maximum value of peak at *-th peak)
exp: exponential function with natural number e as base

The assignment of the peaks marked with * in the above is as follows.

0: peak at 354 $cm^{-1}$ $\pm$ 5 $cm^{-1}$
1: peak at 782 $cm^{-1}$ $\pm$ 5 $cm^{-1}$
2: peak at 820 $cm^{-1}$ $\pm$ 5 $cm^{-1}$
3: peak at 868 $cm^{-1}$ $\pm$ 5 $cm^{-1}$
4: peak at 886 $cm^{-1}$ $\pm$ 5 $cm^{-1}$
5: peak at 900 $cm^{-1}$ $\pm$ 5 $cm^{-1}$
6: peak at 940 $cm^{-1}$ $\pm$ 4 $cm^{-1}$
7: peak at 952 $cm^{-1}$ $\pm$ 7 $cm^{-1}$
8: peak at 968 $cm^{-1}$ $\pm$ 5 $cm^{-1}$
9: peak at 989 $cm^{-1}$ $\pm$ 5 $cm^{-1}$

[R2]

The catalyst according to the present invention is characterized in that R2 represented by the above equation (2) is 951.4 $cm^{-1}$ or more and 959.0 $cm^{-1}$ or less.
R2 is the wave number for giving the maximum value of the peak at 952 $cm^{-1}$ $\pm$ 7 $cm^{-1}$ measured by Raman spectroscopy, and is a parameter obtained as p-7 by fitting the measurement data according to the above equation (4). The present inventors consider that R2 indicates the distance or strength of a bond associated with oxygen atoms in nickel molybdate or cobalt molybdate, as measured by Raman spectroscopy. The present inventors have found that high R2 indicates low activity and a high selectivity since oxygen (bulk lattice oxygen used in oxidation reactions) bonding in nickel molybdate or cobalt molybdate is strong, and conversely lowering R2 corresponds to relaxing of oxygen bonding and this indicates high activity and a low selectivity, so that there is an optimal numerical range for R2.
That is, it has been found that the catalyst according to the present invention can achieve a high selectivity and a high yield when R2 is 951.4 $cm^{-1}$ or more and 959.0 $cm^{-1}$ or less.
The lower limit of R2 is more preferably 951.5 $cm^{-1}$, and is 951.7 $cm^{-1}$, 951.9 $cm^{-1}$, 952.1 $cm^{-1}$, and 952.2 $cm^{-1}$ in order of preference. The upper limit of R2 is more preferably 958.0 $cm^{-1}$, and is 957.0 $cm^{-1}$, 956.0 $cm^{-1}$, 955.0 $cm^{-1}$, 954.0 $cm^{-1}$, 953.0 $cm^{-1}$, 952.8 $cm^{-1}$, and 952.6 $cm^{-1}$ in order of preference. That is, a more preferred range for the value of R2 is set by the above upper and lower limits, and is, for example, 951.5 $cm^{-1}$ or more and 954.0 $cm^{-1}$ or less, and most preferably 952.2 $cm^{-1}$ or more and 952.6 $cm^{-1}$ or less.

[R3]

In the catalyst according to the present invention, R3 represented by the above equation (3) is 0.21 or more. The essence of R3 is based on the point that a result of an improved selectivity is recognized when a certain relationship is recognized between R1 and R2. R3 is a parameter regressed based on the value of R1 and the value of R2 and the selectivity. That is, R3 is considered to show a high numerical value if (R1) the amount of O-Mo bond is large and/or (R2) the bond associated with oxygen atoms in nickel molybdate or cobalt molybdate is strong. The present inventors have found a relationship between the numerical range and the useful yield.
The lower limit of R3 is more preferably 0.30, and is 0.36, 0.40, 0.50, 0.65, 0.70, and 0.80 in order of preference. The upper limit of R3 is not limited, and may be about 2.0, and more preferably about 1.0.

[Catalyst Composition]

A catalytically active component contained in the catalyst according to the present invention preferably has a composition represented by the following formula (I).
[Chem. 2]

$$Mo_{a1}Bi_{b1}Ni_{c1}Co_{d1}Fe_{e1}X_{f1}Y_{g1}Z_{h1}O_{i1} \qquad (I)$$

(In the formula, Mo, Bi, Ni, Co, and Fe respectively represent molybdenum, bismuth, nickel, cobalt, and iron; X is at least one element selected from tungsten, antimony, tin, zinc, chromium, manganese, magnesium, silicon, aluminum, cerium, and titanium; Y is at least one element selected from sodium, potassium, cesium, rubidium, and thallium; Z belongs to Groups 1 to 16 in the periodic table, and means at least one element selected from elements other than the above Mo, Bi, Ni, Co, Fe, X, and Y; a1, b1, c1, d1, e1, f1, g1, h1, and i1 respectively represent the number of atoms of molybdenum, bismuth, nickel, cobalt, iron, X, Y, Z, and oxygen; and when $a1 = 12$, $0 < b1 \leq 7$, $0 \leq c1 \leq 10$, $0 < d1 \leq 10$, $0 < c1+d1 \leq 20$, $0 \leq e1 \leq 5$, $0 \leq g1 \leq 2$, $0 \leq f1 \leq 3$, $0 \leq h1 \leq 5$, and i1 is a value determined by an oxidation state of each element.)

In the above formula (I), when $a1 = 12$, preferred ranges of b1 to i1 are as follows.

The lower limit of b1 is 0.2, 0.5, 0.6, and 0.7 in order of preference, and most preferably 0.8. The upper limit of b1 is 5, 3, 2, 1.6, 1.4, and 1.2 in order of preference, and most preferably 1.1. That is, the most preferred range of b1 is $0.8 \leq b1 \leq 1.1$.

The lower limit of c1 is 1, 1.5, 2.0, 2.2, and 2.4 in order of preference, and most preferably 2.5. The upper limit of c1 is 5, 4, 3.8, 3.6, and 3.4 in order of preference, and most preferably 3.2. That is, the most preferred range of c1 is $2.5 \leq c1 \leq 3.2$.

The lower limit of d1 is 3, 4, 5, 5.3, 5.5, and 5.7 in order of preference, and most preferably 5.8. The upper limit of d1 is 8, 7, 6.5, 6.3, and 6.1 in order of preference, and most preferably 6.0. That is, the most preferred range of d1 is $5.8 \leq d1 \leq 6.0$.

The lower limit of e1 is 0.5, 1, 1.2, and 1.4 in order of preference, and most preferably 1.5. The upper limit of e1 is 4, 3, 2.5, 2.3, and 2.1 in order of preference, and most preferably 2.0. That is, the most preferred range of e1 is $1.5 \leq e1 \leq 2.0$.

The upper limit of f1 is 8, 7, 6, and 5 in order of preference. That is, the most preferred range of f1 is $0 \leq f1 \leq 5$.

The lower limit of g1 is 0, 0.02, 0.04, 0.05, and 0.06 in order of preference, and most preferably 0.07. The upper limit of g1 is 1.5, 1, 0.5, 0.2, and 0.15 in order of preference, and most preferably 0.10. That is, the most preferred range of g1 is $0.07 < g1 \leq 0.10$.

The upper limit of h1 is 8, 7, 6, and 5 in order of preference. That is, the most preferred range of h1 is $0 \leq h1 \leq 5$.

It is preferable that two or less types of Y are contained, and one type of Y is particularly preferred. In a particularly preferred embodiment, f1 and h1 are 0.

[Carrying]

The catalyst in which a preliminary calcined powder subjected to preliminary calcination after the preparation of the catalytically active component is carried on the inert carrier is particularly excellent as the catalyst of the present invention.

As the material of the inert carrier, known materials such as alumina, silica, titania, zirconia, niobia, silica alumina, silicon carbide, carbides, steatite, and mixtures thereof can be used. Further, the particle size, water absorption rate, mechanical strength, crystallinity of each crystal phase, mixing ratio, etc. are not limited, and an appropriate range of these should be selected in consideration of the final catalyst performance, molding properties, production efficiency, etc. The mixing ratio of the carrier and the preliminarily calcined powder is calculated as the active mass ratio according to the following equation based on the charged mass of each raw material.

Active mass ratio (mass%) = (mass of preliminary calcined powder used for molding)/{(mass of preliminary calcined powder used for molding) + (mass of carrier used for molding)} × 100

The upper limit of the active mass ratio is preferably 80 mass%, and more preferably 60 mass%.

The lower limit is preferably 20 mass%, and more preferably 30 mass%. That is, the most preferred range of the active mass ratio is 30 mass% or more and 60 mass% or less.

As the inert carrier, silica and/or alumina is preferable, and a mixture of silica and alumina is particularly preferable.

For the carrying, it is preferred to use a binder. Specific examples of the binder which can be used include water, ethanol, methanol, propanol, a polyhydric alcohol, polyvinyl alcohol as a polymer-based binder, and a silica sol aqueous solution as an inorganic binder; ethanol, methanol, propanol, and a polyhydric alcohol are preferred; a diol such as ethylene glycol and a triol such as glycerin is preferred; and an aqueous solution having a concentration of glycerin of 5 mass% or more is preferred. Using an appropriate amount of a glycerin aqueous solution allows for obtaining a high-performance catalyst having good molding properties and high mechanical strength. The amount of these binders used is usually 2 to 60 parts by mass with respect to 100 parts by mass of the preliminarily calcined powder. The amount of glycerin aqueous solution is preferably 10 to 30 parts by mass. The binder and the preliminarily calcined powder may be alternately or simultaneously supplied to a molding machine on the occasion of carrying.

[Method for Producing Catalyst According to Present Invention]

A method for producing the catalyst according to the present invention includes: (a) a step of dispersing compounds containing each or a plurality of the above metals in water to prepare an aqueous solution or aqueous dispersion of the compounds (hereinafter both are referred to as a slurry liquid); (b) a step of drying the slurry liquid obtained in the step (a) to obtain a slurry dried body; (c) a step of shaping the slurry dried body obtained in the step (b); and (d) a step of calcination a coated shaped product obtained in the step (c), but is not limited thereto.

<Step (a)>

In the step (a), starting raw materials for the elements are not limited, but for example, as the molybdenum component raw material, molybdenum oxides such as molybdenum trioxide, molybdic acid or salts thereof, such as molybdic acid, ammonium paramolybdate, and ammonium metamolybdate, and heteropoly acids containing molybdenum or salts thereof, such as phosphomolybdic acid and silicomolybdic acid, can be used.

As the bismuth component raw material, bismuth salts such as bismuth nitrate, bismuth carbonate, bismuth sulfate, and bismuth acetate, bismuth trioxide, metal bismuth and the like can be used. These raw materials can be used as solids or as an aqueous solution, a nitric acid solution, or a slurry of bismuth compounds generated from these aqueous solutions. It is preferable to use a nitrate, a solution thereof, or a slurry obtained from the solution.

As starting raw materials for other component elements, an ammonium salt, a nitrate, a nitrite, a carbonate, a subcarbonate, an acetate, a chloride, an inorganic acid, an inorganic acid salt, a heteropoly acid, a heteropoly acid salt, a sulfate, a hydroxide, an organic acid salt, and an oxide of metallic elements that are commonly used in this type of catalyst, or a mixture thereof may be used in combination. An ammonium salt and a nitrate are preferably used.

A compound containing these active components may be used alone or in combination of two or more thereof. A slurry liquid can be obtained by uniformly mixing each active component-containing compound and water. The amount of water to be used in the slurry liquid is not limited as long as it can completely dissolve the entire amount of the compound used or can enable uniform mixing. The amount of water to be used may be appropriately determined in consideration of the drying method and drying conditions. Typically, the amount of water to be used is 100 parts by mass or more and 2000 parts by mass or less with respect to 100 parts by mass of the total mass of a slurry preparation compound. The amount of water may be large, but too large amount of water has many disadvantages that the energy cost in the drying step increases, drying cannot be completed and the like.

The slurry liquid of the source compound of each component element is preferably prepared by (a) a method of mixing each of the above source compounds at once, (b) a method of mixing the above slurry liquids and then performing aging, (c) a method of mixing the above source compounds stepwise, (d) a method of repeating mixing step and aging step stepwise, and (e) a method combining (a) to (d). Here, the above aging means "an operation in which industrial raw materials or semi-finished products are treated under specific conditions such as a certain period of time and a certain temperature to acquire or improve the required physical properties and chemical properties, or proceed with a predetermined reaction". In the present invention, the above certain period of time means a range of 5 minutes or longer and 24 hours or shorter, and the above certain temperature means a range equal to or lower than a boiling point of an aqueous solution or an aqueous dispersion liquid and equal to or higher than room temperature. Among these, in terms of the activity and yield of the finally obtained catalyst, preferred is the (c) method of mixing the above source compounds stepwise, more preferred is a method of preparing a completely dissolved solution for each raw material to be mixed with a mother liquid stepwise, and most preferred is a method of mixing various mixed solutions of an alkali metal solution and a nitrate with a mother liquid made from the molybdenum raw material as a blended solution or slurry. However, it is not always necessary to mix all the catalyst constituent elements in this step, and some elements or some amounts thereof may be added in the subsequent steps.

In the present invention, the shape of stirring blades of a stirrer to be used in mixing essential active component is not limited, and any stirring blades such propeller blades, turbine blades, paddle blades, inclined paddle blades, screw blades, anchor blades, ribbon blades, and large lattice blades can be used in one stage or two or more stages of the same type of blade or different types of blades in the up-down direction. In addition, a baffle (baffle plate) may be installed in a reaction vessel as required.

[pH Adjustment]

A particularly preferred production method for obtaining the catalyst according to the present invention is a method including, in the step (a), a blending step of adding each of a source compound containing molybdenum, a source compound containing bismuth and a source compound containing iron to a solvent or solution and performing integration and heating to form a blended solution, in which the pH of the blended solution is adjusted to a range of 1.0 to 7.5 before adding the iron raw material. Examples of a pH adjustment method include a method of lowering the pH by adding nitric acid or a method of raising the pH by adding ammonia water or the like, which will be described in detail later.

The lower limit of the pH is more preferably 1.5, and is 1.8, 2.0, 2.2, 2.5, 2.7, 3.0, 3.1, 3.2, 3.5, 3.7, 3.8, and 3.9 in order of preference. In addition, the upper limit of the pH is more preferably 7.3, and is 7.0, 6.7, 6.5, 6.3, 6.1, 6.0, 5.9, 5.8, 5.5, 5.3, 5.0, 4.8, and 4.5 in order of preference. That is, a more preferred range for the value of the pH is set by the above upper and lower limits, and is, for example, 1.5 or more and 7.3 or less, and most preferably 3.9 or more and 4.5 or less. In the present invention, the pH may fall within the above range even without adding a pH adjusting agent before adding the iron raw material, but the essence of the present invention is to adjust the pH within the above range by adding a pH adjusting agent before adding the iron raw material.

In the method of lowering the pH, examples of the pH adjusting agent include acids such as nitric acid, sulfuric acid, hydrochloric acid, and oxalic acid, which are typically used as catalyst raw materials by those skilled in the art for pH adjustment, and pH adjusting agents such as phosphoric acid, boric acid, molybdic acid, and iron nitrate, whose elements remain after calcination as long as they do not change the elemental composition of the catalytically active component to be described later, and nitric acid is most preferred. In the method of raising the pH, examples of the pH adjusting agent include ammonia water, pyridine, and an ammonium carbonate aqueous solution, which are typically used as catalyst raw materials by those skilled in the art for pH adjustment, and pH adjusting agents such as potassium hydroxide and cesium hydroxide, whose elements remain after calcination as long as they do not change the elemental composition of the catalytically active component to be described later, and ammonia water is most preferred.

In the case of performing this method, the most preferred embodiment is a method of preparing a slurry in the order of adding the iron raw material and then adding the bismuth raw material to the blended solution after stirring.

In the case of dropping the pH adjusting agent, the agent is added while stirring at a stirring power of 0.01 $kw/m^3$ to 5.00 $kw/m^3$. The lower limit of the stirring power is preferably 0.05 $kw/m^3$, 0.10 $kw/m^3$, 0.50 $kw/m^3$, and 1.00 $kw/m^3$, and the upper limit of the stirring power is preferably 4.50 $kw/m^3$, 4.00 $kw/m^3$, 3.50 $kw/m^3$, and 3.00 $kw/m^3$. That is, the most preferred range of the stirring power is 1.00 $kw/m^3$ to 3.00 $kw/m^3$.

The dropping time for the pH adjusting agent is 1 second to 5 minutes. The lower limit of the dropping time is preferably 5 seconds, 10 seconds, and 15 seconds, and the upper limit of the dropping time is preferably 4 minutes, 3 minutes, 2 minutes and 30 seconds, 2 minutes, 1 minute and 30 seconds, 1 minute, 45 seconds, and 30 seconds. That is, the most preferred range of the dropping time is 15 seconds to 30 seconds.

The solution temperature of the blended solution at the time of dropping the pH adjusting agent is 5°C to 80°C. The lower limit of the solution temperature is preferably 10°C, 20°C, and 30°C, and the upper limit of the solution temperature is preferably 70°C, 60°C, 50°C, and 40°C. That is, the most preferred range of the solution temperature is 30°C to 40°C.

<Step (b)>

In the step (b), the slurry liquid obtained in the step (a) is dried. The drying method is not limited as long as it is a method capable of completely drying the slurry liquid, and examples thereof include drum drying, freeze drying, spray drying, and evaporation to dryness. Among these, in the present invention, spray drying is particularly preferred because the slurry liquid can be dried into a powder or granules in a short period of time. The drying temperature in the spray drying varies depending on the concentration of the slurry liquid, the liquid feeding rate, or the like, and the temperature at the outlet of the dryer is typically 70°C or higher and 150°C or lower.

<Step (c)>

In the step (c), the slurry dried body obtained in the step (b) is shaped. Calcination may be performed before shaping, though it is not essential. In the present description, calcination before shaping is referred to as preliminary calcination.

As the shaping, both shaping methods of carrying shaping in which a carrier such as silica is carried and non-carrying shaping without using a carrier can be used. Specific shaping methods include tablet shaping, press shaping, extrusion shaping, and granulation shaping. As the shape of the shaped product, for example, a columnar shape, a ring shape, a spherical shape, or the like can be appropriately selected in consideration of operating conditions. Preferred is a carried catalyst in which a catalytically active component is carried on a spherical carrier, particularly an inert carrier such as silica or alumina, having an average particle size of 3.0 mm or more and 10.0 mm or less, and preferably having an average particle size of 3.0 mm or more and 8.0 mm or less. As for the carrying method, a tumbling granulation, a method using a centrifugal flow coating apparatus, a wash coating, and the like are widely known, and the method is not limited as long as the preliminarily calcined powder can be uniformly carried on the carrier. The tumbling granulation is preferred in consideration of the production efficiency of the catalyst and the like. Specifically, this method is a method of rotating a flat or uneven disc at a high speed in an apparatus that has the disc at the bottom of a fixed cylindrical container, to vigorously agitate a carrier charged in the container by repeating the rotation and revolution movements of the carrier, and carrying the powder component on the carrier by adding the preliminarily calcined powder to the container. It is preferable to use a binder for carrying. Specific examples of the binder which can be used include water, ethanol, methanol, propanol, a polyhydric alcohol, polyvinyl alcohol that is a polymer-based binder, and a silica sol aqueous solution that is an inorganic binder. Ethanol, methanol, propanol, and a polyhydric alcohol are preferred, a diol such as ethylene glycol and a triol such as glycerin are more preferred, and an aqueous solution having a concentration of glycerin of 5 mass% or more is still more preferred. Using an appropriate amount of a glycerin aqueous solution allows for providing a high-performance catalyst having good shaping properties and high mechanical strength. The amount of these binders to be used is typically 2 to 60 parts by mass with respect to 100 parts by mass of the preliminarily calcined powder, and 15 to 50 parts by mass is preferred in the case of using a glycerin aqueous solution. The binder and the preliminarily calcined powder may be alternately supplied to a shaping machine or simultaneously supplied to the shaping machine during carrying. Further, in the shaping, a small amount of known additives such as graphite and talc may be added. None of a shaping aid, a pore-forming agent, and a carrier added during the shaping is considered as the constituent element of the active component in the present invention, regardless of having activity in the sense of converting the raw material into some other product.

<Step (d)>

In the step (d), the shaped dried body or coated catalyst of the step (b) obtained in the step (c) is calcined. In the present description, the step (d) is expressed as main calcination.

The dried body or coated catalyst can be directly supplied as a catalyst for the catalytic gas phase oxidation reaction, but the main calcination is preferred because it stabilizes the structure and improves the catalyst performance.

The preliminary calcination method, the preliminary calcination conditions, the main calcination method, and the main calcination conditions are not limited, and known treatment methods and conditions can be applied. The preliminary calcination or the main calcination is typically carried out at 200°C or higher and 600°C or lower, and preferably 300°C or higher and 550°C or lower, for 0.5 hours or longer, and preferably 1 hour or longer and 40 hour or shorter under a flow of an oxygen-containing gas such as air or a flow of an inert gas. Here, the inert gas refers to a gas that does not reduce the reaction activity of the catalyst, and specific examples thereof include nitrogen, carbon dioxide, helium, and argon. The optimum conditions for particularly the main calcination differ depending on the reaction conditions at the time of producing an unsaturated aldehyde and/or an unsaturated carboxylic acid using a catalyst, and it is known to those skilled in the art to change step parameters of the main calcination step, such as the oxygen content in the atmosphere, the maximum temperature and the calcination time. Thus, the above change falls within the scope of the present invention. In addition, the main calcination step shall be carried out after the above preliminary calcination step, and the maximum temperature (main calcination temperature) in the main calcination step is higher than the maximum temperature (preliminary calcination temperature) in the above preliminary calcination step. The calcination method is not limited to a fluidized bed, rotary kiln, muffle furnace, tunnel calcination furnace, etc., and an appropriate range should be selected in consideration of the final catalyst performance, mechanical strength, shaping properties, production efficiency, etc.

The catalyst according to the present invention is preferably used as a catalyst for producing an unsaturated aldehyde compound and an unsaturated carboxylic acid compound, is more preferably used as a catalyst for producing an unsaturated aldehyde compound, and is particularly preferably used as a catalyst for producing acrolein from propylene. In an exothermic reaction process such as the production of an unsaturated aldehyde compound and an unsaturated carboxylic acid compound, it is known to those skilled in the art to fill different kinds of catalysts in two or more layers for increasing the activity from an inlet side of a reaction tube to an outlet side of the reaction tube, for the purpose of preventing the deterioration of the catalyst itself due to the heat generated by the reaction in an actual plant. The catalyst according to the present invention can be used on one of the inlet side of the reaction tube, the outlet side of the reaction tube, and an intermediate catalyst layer, and is most preferably used, for example, on the most outlet side of the reaction tube, i.e., used for the most active catalyst among all catalyst layers in the reaction tube. In multi-layer filling, two-layer or three-layer filling is a particularly preferred embodiment. In addition, it is known to those skilled in the art that the catalyst to be used as the catalyst for producing an unsaturated aldehyde compound and an unsaturated carboxylic acid compound can also be applied to a catalyst for producing a conjugated diolefin by an oxidative dehydrogenation reaction from a monoolefin raw material having 4 or more carbon atoms, specifically, a catalyst for producing butadiene by an oxidative dehydrogenation reaction from a n-butene raw material, and such an application is also possible.

[Adjustment Method for R1, R2, and R3]

The values of the above R1, R2, and R3 can be controlled by the pH adjustment method in the step (a), and can also be controlled by other methods such as (I) a method of changing the catalyst composition, (II) a method of changing calcination conditions, (III) a method of changing temperature-lowering conditions after calcination, (IV) a method of controlling a catalyst and a precursor thereof so as not to apply mechanical strength in all steps of catalyst production, (V) a method of using a raw material having high purity, other methods of (VI) to (VIII), and a method of combining (I) to (VIII).

<Method (I)>

Examples of the method (I) include, in the above formula (I), a method of changing the catalyst composition such that

the upper limit of $e1/b1$ is set to 3.30, and preferably 3.00, and the lower limit of $e1/b1$ is set to 0.10, 0.50, 1.00, 1.40, and 1.50 in order of preference,
the upper limit of $d1/b1$ is set to 9.5, 9.0, 8.5, and 8.0 in order of preference, and the lower limit of $d1/b1$ is set to 2.0, 3.0, 4.0, 5.0, and 5.5 in order of preference,
the upper limit of $c1/e1$ is set to 4.0, 3.0, and 2.5 in order of preference, and the lower limit of $c1/e1$ is set to 1.0, 1.1, and 1.2 in order of preference,
the upper limit of $c1/d1$ is set to 2.0, 1.0, and 0.8 in order of preference, and the lower limit of $c1/d1$ is set to 0.3 and 0.4 in order of preference,
the upper limit of $g1/d1$ is set to 0.10, 0.05, 0.04, and 0.03 in order of preference, and the lower limit of $g1/d1$ is set to 0.01, and
the upper limit of $g1/c1$ is set to 0.041 and 0.039 in order of preference, and the lower limit of $g1/c1$ is set to 0.020, 0.025, and 0.027 in order of preference.

<Method (II)>

Examples of the method (II) include a method of changing calcination conditions such that, in the preliminary calcination, the main calcination, or both, the temperature is set to 200°C or higher and 600°C or lower, preferably 300°C or higher and 550°C or lower, and more preferably 460°C or higher and 550°C or lower, the time is set to 0.5 hours or longer, preferably 1 hour or longer and 40 hours or shorter, more preferably 2 hours or longer and 15 hours or shorter, and most preferably 2 hours or longer and 9 hours or shorter, and the atmosphere has an oxygen concentration of 10 vol% or more and 40 vol% or less, preferably 15 vol% or more and 30 vol% or less, and most preferably an air atmosphere.

<Method (III)>

Examples of the method (III) include a method of changing temperature-lowering conditions after calcination such that, in the preliminary calcination, the main calcination, or both, a temperature decrease rate (temperature-lowering rate) on the catalyst surface from the maximum temperature (preliminary calcination temperature or main calcination temperature) during the calcination step to room temperature is set to 1°C/min or more and 200°C/min or less, preferably 5°C/min or more and 150°C/min or less, more preferably 10°C/min or more and 120°C/min or less, and most preferably 50°C/min or more and 100°C/min or less. Temperature lowering methods to be typically used industrially in order to achieve the above temperature-lowering rate range, for example, a method of exposing the catalyst taken out of a calcination furnace to an inert atmosphere or mist of an inert solvent, and a method of rapidly moving the catalyst into a sufficiently pre-cooled chamber are all within the scope of the present embodiment.

<Method (IV)>

Examples of the method (IV) include a method of controlling a catalyst precursor and/or granules formed in each step so as not to apply mechanical impact and shear stress. The control is made such that a preferred range of the mechanical impact and shear stress is set to 100 kgf or less, preferably 50 kgf or less, more preferably 20 kgf or less, still more preferably 10 kgf or less, and most preferably 5 kgf or less.

<Method (V)>

Regarding the method (V), the details are not limited as long as it is a method of using a reagent-grade high-purity raw material. Examples thereof include a method of using a high-purity raw material in which, for example, the content of sulfur and a compound thereof, lithium, a halogen and compounds thereof, and lead is 10000 ppm by weight or less, preferably 1000 ppm by weight or less, more preferably 100 ppm by weight, and most preferably 10 ppm by weight or less.

<Method (VI)>

Examples of the method (VI) include a method of once obtaining a catalyst precursor as granules and shaping the granules. Obtaining the catalyst precursor as granules allows for production of catalysts in which each component of the catalyst is more uniformed.

<Method (VII)>

Examples of the method (VII) include a method of controlling a time of mixing, reacting, slurrying and staying of the cobalt raw material and the nickel raw material in a blending vessel, to be as short as possible in the catalyst blending step, more specifically, a method of shortening the staying time in a situation where there are no metal salt raw materials other than molybdenum and alkali metals but the cobalt raw material and the nickel raw material in the blending vessel, or a method of shortening the staying time in a situation where there are the cobalt raw material and the nickel raw material when the pH in the blending vessel is in a specific range. The staying time is preferably 24 hours, more preferably 1 hour, still more preferably 30 minutes, and most preferably 10 minutes. The above pH range is 1 or more and 14 or less, preferably 2 or more and 10 or less, more preferably 2 or more and 8 or less, and most preferably 3 or more and 7 or less. The same applies to the iron raw material and the bismuth raw material, and the molybdenum raw material and the bismuth raw material.

<Method (VIII)>

Examples of the method (VIII) include a method of charging each raw material at once in the blending step undividedly or a method of reducing a nitric acid concentration in the blended solution, in the catalyst blending step to be described later. The above method of charging at once means charging a next raw material after all the required amount of a raw material have been charged. Regarding the nitric acid concentration in the blended solution, a nitrate ion concentration in terms of mass% is preferably 40 mass% or less, more preferably 35 mass% or less, still more preferably 30 mass% or less, and most preferably 25 mass% or less, in a blended solution at the time when the blending is completed and proceeds to the next step.

< Catalyst in Second Stage>

When the catalyst of the present invention is used as a catalyst in a first stage, that is, a catalyst for producing an unsaturated aldehyde compound, an unsaturated carboxylic acid compound can be obtained by performing a second-stage oxidation reaction.

In this case, the catalyst of the present invention can also be used as a catalyst in a second stage, but a catalyst containing a catalytically active component represented by the following formula (II) is preferred.
[Chem 3]

$$Mo_{12}V_{a2}Wb_2Cu_{c2}Sb_{d2}X2_{e2}Y2_{f2}Z2_{g2}O_{h2}...\qquad(II)$$

(In the formula, Mo, V, W, Cu, Sb and O represent molybdenum, vanadium, tungsten, copper, antimony and oxygen, respectively; X2 represents at least one element selected from the group consisting of an alkali metal and thallium; Y2 represents at least one element selected from the group consisting of magnesium, calcium, strontium, barium and zinc; and Z2 represents at least one element selected from the group consisting of niobium, cerium, tin, chromium, manganese, iron, cobalt, samarium, germanium, titanium and arsenic. a2, b2, c2, d2, e2, 12, g2 and h2 represent the atomic proportion of each element, and with respect to molybdenum atom 12, a2 satisfies $0 < a2 \le 10$, b2 satisfies $0 \le b2 \le 10$, c2 satisfies $0 < c2 \le 6$, d2 satisfies $0 < d2 \le 10$, e2 satisfies $0 \le e2 \le 0.5$, f2 satisfies $0 \le f2 \le 1$, and g2 satisfies $0 \le g2 < 6$. Further, h2 is the number of oxygen atoms required to satisfy the atomic value of each component.)

In the production of a catalyst containing the catalytically active component represented by the above formula (II), a method widely known as a method for preparing this kind of a catalyst, for example, an oxide catalyst or a catalyst having a heteropolyacid or a salt structure thereof, can be adopted. The raw materials that can be used in producing the catalyst are not limited, and various materials can be used. For example, molybdenum oxides such as molybdenum trioxide, molybdic acid or salts thereof such as molybdic acid and an ammonium molybdate, molybdenum-containing heteropolyacids or salts thereof such as phosphomolybdic acid and silicomolybdic acid, and the like can be used. The raw material of an antimony component is not limited, but antimony trioxide or antimony acetate is preferred. As raw materials for other elements such as vanadium, tungsten, copper and the like, nitrate, sulfate, carbonate, phosphate, organic acid salt, halide, hydroxide, oxide or the metal of these elements can be used. A compound containing these active components may be used alone or in combination of two or more.

Next, the slurry liquid obtained above is dried to obtain a solid of catalytically active component. The drying method is not limited so long as the slurry liquid can be completely dried by the method. However, examples thereof include drum drying, freeze drying, spray drying and evaporation drying. Spray drying is preferred because the slurry liquid can be dried into a powder or granule in a short period of time. The temperature of the drying with spray drying varies depending on the concentration of the slurry liquid, the liquid sending speed or the like, but the temperature at the outlet of a drying machine is approximately 70°C to 150°C. In this case, the slurry liquid is preferably dried such that the average particle size of a slurry liquid dried product (catalyst precursor) to be obtained is 10 μm to 700 μm.

The solid of catalytically active component in the second stage obtained as described above can be used as it is for a coating mixture, and is preferably subjected to calcination because the molding properties may be improved. The calcination method and the calcination conditions are not limited, and known treatment methods and conditions can be applied. The optimum calcination conditions vary depending on the used raw material for the catalyst, catalyst composition, preparation method and the like. The calcination temperature is usually 100°C to 350°C, preferably 150°C to 300°C, and the calcination time is usually 1 to 20 hours. The calcination is usually carried out in an air atmosphere, but may be carried out in an atmosphere of an inert gas such as nitrogen, carbon dioxide, helium or argon. The calcination in an air atmosphere may be carried out after calcination in an inert gas atmosphere, if necessary. The thus-obtained calcined solid is preferably pulverized before the molding. The pulverizing method is not limited, but it is preferable to use a ball mill.

The compound containing the active component in preparing the slurry for the second stage does not necessarily have to contain all the active components, and a part of the components may be used before the following molding step.

The shape of the catalyst in the second stage is not limited. The catalyst is used by being molded into a columnar shape, a tablet, a ring shape, a spherical shape or the like in order to reduce the pressure loss of a reaction gas in the oxidation reaction. Among these, the solid of catalytically active component is particularly preferably carried on an inert carrier to be a carried catalyst because improvement in selectivity and removal of heat of reaction can be expected. A tumbling granulation method described below is preferred for the carrying. This method is a method in which, for example, in a device that has a flat or uneven disk at the bottom of a fixed container, a carrier in the container is vigorously agitated by repeatedly performing rotation motion and revolution motion by rotating the disk at a high speed, and then a mixture for the carrying including the binder, the solid of catalytically active component and optionally a molding aid and/or a strength improver is carried on the carrier. As a method of adding the binder, any methods may be adapted such as 1) premixing a binder with the mixture for the carrying, 2) adding the binder at the same time as the mixture for the carrying is added into the fixed container, 3) adding the binder after adding the mixture for the carrying into the fixed container, 4) adding the binder before adding the mixture for the carrying into the fixed container, 5) dividedly preparing the mixture for the carrying and the binder independently and adding the whole amount of them in the appropriate combination of 2) to 4). Among these, for example, 5) is preferably performed by adjusting the adding rate using an auto feeder or the like such that the mixture for the carrying does not adhere to the wall of the fixed container and the mixture for the carrying does not aggregate with each other and a predetermined amount of the mixture for the carrying is carried on the carrier. Examples of the binder include water, ethanol, polyhydric alcohol, polyvinyl alcohol as a polymer-based binder, celluloses such as a crystalline cellulose, methyl cellulose and ethyl cellulose, and an aqueous silica sol solution as an inorganic binder. Diols such as cellulose and ethylene glycol and triols such as glycerin are preferred, and an aqueous solution having a concentration of glycerin of 5 mass% or more is particularly preferred. The amount of these binders to be used is usually 2 to 60 parts by mass, preferably 10 to 50 parts by mass, per 100 parts by mass of the mixture for the carrying.

Specific examples of the carrier in the above carrying include a spherical carrier having a diameter of 1 mm to 15 mm, and preferably 2.5 mm to 10 mm, such as silicon carbide, alumina, silica alumina, mullite and arandom. The carriers having a porosity of 10% to 70% are usually used. The carrier and the mixture for the carrying at the ratio of the mixture for the carrying/(mixture for the carrying + carrier) =10 mass% to 75 mass% are usually used, and the carrier and the mixture for the carrying at the ratio of the mixture for the carrying/(mixture for the carrying + carrier) =15 mass% to 60 mass% are preferably used. When the ratio of the mixture for the carrying tends to be large, the reaction activity of the carried catalyst is large, but the mechanical strength tends to be small. On the contrary, when the ratio of the mixture for the carrying is small, the mechanical strength tends to be large, but the reaction activity tends to be small. In the above, examples of the molding aid to be used as necessary include silica gel, diatomite, and alumina powder. The amount of the molding aid to be used is usually 1 to 60 parts by mass with respect to 100 parts by mass of the solid of catalytically active component. If necessary, the use of inorganic fibers (for example, ceramic fibers or whiskers) that are inactive to the solid of catalytically active component and the reaction gas as the strength improver is useful for improving the mechanical strength of the catalyst, and glass fibers are preferred. The amount of the fiber to be used is usually 1 to 30 parts by mass with respect to 100 parts by mass of the solid of catalytically active component. None of a molding aid, a pore-forming agent and a carrier added in the molding of the catalyst for the first stage is considered as the constituent element of the active component in the present invention, regardless of whether the molding aid, the pore-forming agent and the carrier have the activity in the sense of converting the raw material into some other product.

The carried catalyst obtained as described above can be used as a catalyst for the catalytic gas phase oxidation, and is preferably subjected to calcination because the molding properties may be improved. The calcination method and the calcination conditions are not limited, and known treatment methods and conditions can be applied. The optimum calcination conditions vary depending on the raw material for the catalyst to be used, the catalyst composition, the preparation method, and the like, but the calcination temperature is usually 100°C to 450°C, preferably 270°C to 420°C, and the calcination time is usually 1 to 20 hours. The calcination is usually carried out in an air atmosphere, and may be carried out in an atmosphere of an inert gas such as nitrogen, carbon dioxide, helium or argon. The calcination in an air atmosphere may be carried out after the calcination in an inert gas atmosphere, if necessary.

When the catalyst of the present invention is used in a reaction of using propylene, isobutylene, t-butyl alcohol and the like as raw materials to produce the corresponding unsaturated aldehyde, unsaturated carboxylic acid, and particularly, in a reaction of producing acrolein and acrylic acid by catalytic gas phase oxidation of propylene with molecular oxygen or a gas containing molecular oxygen, using the catalyst of the present embodiment allows for improving the catalytic activity and the yield, and is very effective in improving the price competitiveness of the product as compared with the known method. In addition, the effect of improving the process stability of the partial oxidation reaction accompanied by heat generation, such as reduction of the hot spot temperature can be expected. Further, the catalyst of the present invention is also effective in reducing by-products that adversely influences the environment and the quality of the final product, such as carbon monoxide (CO), carbon dioxide ($CO_2$), acetaldehyde, acetic acid, and formaldehyde.

The thus-obtained catalyst of the present invention can be used, for example, for producing acrolein and/or acrylic acid by catalytic gas phase oxidation of propylene using a molecular oxygen-containing gas. In the production method of the present invention, the method for flowing the raw material gas may be an ordinary single-flow method or a recycling method, and can be carried out under widely used conditions, and is not limited. For example, a mixed gas containing 1 vol% to 10 vol% and preferably 4 vol% to 9 vol% of propylene, 3 vol% to 20 vol% and preferably 4 vol% to 18 vol% of molecular oxygen, 0 vol% to 60 vol% and preferably 4 vol% to 50 vol% of water vapor at room temperature as a starting raw material, and 20 vol% to 80 vol% and preferably 30 vol% to 60 vol% of an inert gas such as carbon dioxide and nitrogen is introduced to the catalyst of the present invention filled in a reaction tube at 250°C to 450°C under normal pressure to 10 atm and a space velocity of 300 to 5000 $h^{-1}$ to perform a reaction.

In the present invention, unless otherwise specified, the improvement of the catalytic activity means that the conversion rate of the raw material is high when the catalytic reaction is carried out at the same temperature of the reaction bath.

In the present invention, unless otherwise specified, a high yield means that the total yield of the corresponding unsaturated aldehyde and/or unsaturated carboxylic acid is high when the oxidation reaction is performed using propylene, isobutylene, t-butyl alcohol, and the like as raw materials. Unless otherwise specified, the yield refers to an useful yield described later.

In the present invention, unless otherwise specified, the constituent elements of the catalytically active component refer to all the elements to be used in the method for producing a catalyst, but the raw materials and the constituent elements thereof that disappear, sublimate, volatilize, and burn at the maximum temperature or lower in the main calcination step are not included in the constituent elements of the catalytically active component. Further, silicon and the other elements constituting inorganic materials contained in the molding aid and the carrier in the shaping step are not included in the constituent elements of the catalytically active component.

In the present invention, the hot spot temperature refers to the maximum temperature in the temperature distribution in the catalyst-filled bed that is measured in thermocouples installed in the multi-tube reaction tube in the long axis direction, and the temperature of the reaction bath refers to a set temperature of a heat medium used for the purpose of cooling the heat generated in the reaction tube. The number of measuring points in the temperature distribution is not limited, but for example, the catalyst filling length is evenly divided from 10 to 1000.

In the present invention, the unsaturated aldehyde and the unsaturated aldehyde compound refers to organic compounds having at least one double bond and at least one aldehyde in the molecule, such as acrolein and methacrolein. In the present invention, the unsaturated carboxylic acid and the unsaturated carboxylic acid compound refers to organic compounds having at least one double bond and at least one carboxy group or an ester group of the carboxyl group in the molecule, and are, for example, acrylic acid, methacrylic acid, and methyl methacrylate. In the present invention, the conjugated diene refers to a diene in which a double bond is separated by one single bond and which is chemically conjugated, and is, for example, 1,3-butadiene.

The method for producing the catalyst according to the present invention makes it possible to produce the catalyst more simply than the catalyst in the related art, and thus the method allows for obtaining a high-performance catalyst at a low production cost. In addition, the catalyst according to the present invention is effective in improving the yield even in a region where the catalyst activity is not high, and also has an effect of improving the process stability of the partial oxidation reaction accompanied by heat generation, such as reduction of ΔT (difference between hot spot temperature and salt bath temperature).

EXAMPLES

Hereinafter, the present invention will be described in more detail with reference to Examples. In Examples, the raw material conversion rate, the useful yield, the useful selectivity, and the active mass ratio were calculated according to the following equations.

Raw material conversion rate (%) = (number of moles of propylene reacted)/(number of moles of propylene supplied) × 100

Useful yield (%) = (total number of moles of acrolein and acrylic acid produced)/(number of moles of propylene supplied) × 100

Useful selectivity (%) = (total number of moles of acrolein and acrylic acid produced)/(number of moles of propylene reacted) × 100

Active mass ratio (mass%) = (mass of preliminarily calcined powder for use in shaping)/{(mass of preliminarily calcined powder for use in shaping) + (mass of carrier for use in shaping)] × 100

[Example 1]

In 380 parts by mass of pure water heated to 60°C, 100 parts by mass of ammonium heptamolybdate was completely dissolved (mother liquid 1). Next, 0.22 parts by mass of potassium nitrate was dissolved in 1.9 parts by mass of pure water, and the solution was added to the mother liquid 1. Next, 9.3 parts by mass of nitric acid (60 mass%) was added to the mother liquid 1 to adjust the pH to 4. Next, 38 parts by mass of ferric nitrate, 91 parts by mass of cobalt nitrate, and 30 parts by mass of nickel nitrate were dissolved in 84 parts by mass of pure water heated to 60°C, and the solution was added to the mother liquid 1. Subsequently, 16 parts by mass of bismuth nitrate was dissolved in a nitric acid aqueous solution prepared by adding 4.1 parts by mass of nitric acid (60 mass%) to 17 parts by mass of pure water heated to 60°C, and the solution was added to the mother liquid 1. This mother liquid 1 was dried by a spray drying, and the obtained dry powder was subjected to preliminary calcination under conditions of 440°C and 4 hours. To the thus-obtained preliminarily calcined powder (atomic ratio calculated based on the charged raw materials: Mo:Bi:Fe:Co:Ni:K = 12:0.7:2.0:6.6:2.2:0.05), a crystalline cellulose was added in an amount of 5 mass% with respect to the preliminarily calcined powder, and the mixture was subjected to thorough mixing. Then, the resultant mixture was carried and shaped in a spherical shape at an active mass ratio of 50 mass% on an inert carrier by using a 33 mass% glycerin solution as a binder by a tumbling granulation. The thus-obtained spherical shaped product having a particle size of 5.3 mm was subjected to main calcination under conditions of 530°C and 4 hours to obtain a catalyst 1.

[Example 2]

In 380 parts by mass of pure water heated to 60°C, 100 parts by mass of ammonium heptamolybdate was completely dissolved (mother liquid 1). Next, 0.27 parts by mass of potassium nitrate was dissolved in 2.4 parts by mass of pure water, the solution was added to the mother liquid 1. Next, 19.3 parts by mass of ammonia water (10 mass%) was added to the mother liquid 1 to adjust the pH to 6. Next, 44 parts by mass of ferric nitrate, 89 parts by mass of cobalt nitrate, and 32 parts by mass of nickel nitrate were dissolved in 87 parts by mass of pure water heated to 60°C, the solution was added to the mother liquid 1. Subsequently, 16 parts by mass of bismuth nitrate was dissolved in a nitric acid aqueous solution prepared by adding 4.1 parts by mass of nitric acid (60 mass%) to 17 parts by mass of pure water heated to 60°C, and the solution was added to the mother liquid 1. This mother liquid 1 was dried by a spray drying, and the obtained dry powder was subjected to preliminary calcination under conditions of 440°C and 4 hours. To the thus-obtained preliminarily calcined powder (atomic ratio calculated based on the charged raw materials: Mo:Bi:Fe:Co:Ni:K = 12:0.7:2.3:6.5:2.3:0.06), a crystalline cellulose was added in an amount of 5 mass% with respect to the preliminarily calcined powder, and the mixture was subjected to thorough mixing. Then, the resultant mixture was carried and shaped in a spherical shape at an active mass ratio of 50 mass% on an inert carrier by using a 33 mass% glycerin solution as a binder by a tumbling granulation. The thus-obtained spherical shaped product having a particle size of 5.3 mm was subjected to main calcination under conditions of 530°C and 4 hours to obtain a catalyst 2.

[Example 3]

In 380 parts by mass of pure water heated to 60°C, 100 parts by mass of ammonium heptamolybdate was completely dissolved (mother liquid 1). Next, 0.42 parts by mass of potassium nitrate was dissolved in 3.8 parts by mass of pure water, and the solution was added to the mother liquid 1. Next, 10.6 parts by mass of nitric acid (60 mass%) was added to the mother liquid 1 to adjust the pH to 4. Next, 38 parts by mass of ferric nitrate, 82 parts by mass of cobalt nitrate, and 40 parts by mass of nickel nitrate were dissolved in 85 parts by mass of pure water heated to 60°C, the solution was added to the mother liquid 1. Subsequently, 17 parts by mass of bismuth nitrate was dissolved in a nitric acid aqueous solution prepared by adding 4.4 parts by mass of nitric acid (60 mass%) to 18 parts by mass of pure water heated to 60°C, and the solution was added to the mother liquid 1. This mother liquid 1 was dried by a spray drying, and the obtained dry powder was subjected to preliminary calcination under conditions of 440°C and 4 hours. To the thus-obtained preliminarily calcined powder (atomic ratio calculated based on the charged raw materials: Mo:Bi:Fe:Co:Ni:K = 12:0.8:2.0:6.0:2.9:0.09), a crystalline cellulose was added in an amount of 5 mass% with respect to the preliminarily calcined powder, the mixture was subjected to thorough mixing. Then, the resultant mixture was carried and shaped in a spherical shape at an active mass ratio of 50 mass% on an inert carrier by using a 33 mass% glycerin solution as a binder by a tumbling granulation. The thus-obtained spherical shaped product having a particle size of 5.3 mm was subjected to main calcination under conditions of 530°C and 4 hours to obtain a catalyst 3.

[Example 4]

In 380 parts by mass of pure water heated to 60°C, 100 parts by mass of ammonium heptamolybdate was completely dissolved (mother liquid 1). Next, 0.31 parts by mass of potassium nitrate was dissolved in 2.9 parts by mass of pure water, and the solution was added the mother liquid 1. Next, 10.6 parts by mass of nitric acid (60 mass%) was added to the mother liquid 1 to adjust the pH to 4. Next, 35 parts by mass of ferric nitrate, 84 parts by mass of cobalt nitrate, and 36 parts by mass of nickel nitrate were dissolved in 82 parts by mass of pure water heated to 60°C, and the solution was added to the mother liquid 1. Subsequently, 17 parts by mass of bismuth nitrate was dissolved in a nitric acid aqueous solution prepared by adding 4.4 parts by mass of nitric acid (60 mass%) to 18 parts by mass of pure water heated to 60°C, and the solution was added to the mother liquid 1. This mother liquid 1 was dried by a spray drying, and the obtained dry powder was subjected to preliminary calcination under conditions of 440°C and 4 hours. To the thus-obtained preliminarily calcined powder (atomic ratio calculated based on the charged raw materials: Mo:Bi:Fe:Co:Ni:K = 12:0.8:1.9:6.1:2.6:0.07), a crystalline cellulose was added in an amount of 5 mass% with respect to the preliminarily calcined powder, and the mixture was subjected to thorough mixing. Then, the resultant mixture was carried and shaped in a spherical shape at an active mass ratio of 50 mass% on an inert carrier by using a 33 mass% glycerin solution as a binder by a tumbling granulation. The thus-obtained spherical shaped product having a particle size of 5.3 mm was subjected to main calcination under conditions of 520°C and 4 hours to obtain a catalyst 4.

[Comparative Example 1]

In 380 parts by mass of pure water heated to 60°C, 100 parts by mass of ammonium heptamolybdate was completely dissolved (mother liquid 1). Next, 0.17 parts by mass of potassium nitrate was dissolved in 1.9 parts by mass of pure water, and the solution was added to the mother liquid 1. The pH of the mother liquid 1 at this time was 5.2. Next, 41 parts by mass of ferric nitrate, 89 parts by mass of cobalt nitrate, and 33 parts by mass of nickel nitrate were dissolved in 85 parts by mass of pure water heated to 60°C, the solution was added to the mother liquid 1. Subsequently, 16 parts by mass of bismuth nitrate was dissolved in a nitric acid aqueous solution prepared by adding 4.1 parts by mass of nitric acid (60 mass%) to 17 parts by mass of pure water heated to 60°C, and the solution was added to the mother liquid 1. This mother liquid 1 was dried by a spray drying, and the obtained dry powder was subjected to preliminary calcination under conditions of 440°C and 4 hours. To the thus-obtained preliminarily calcined powder (atomic ratio calculated based on the charged raw materials: Mo:Bi:Fe:Co:Ni:K = 12:0.7:2.2:6.5:2.4:0.04), a crystalline cellulose was added in an amount of 5 mass% with respect to the preliminarily calcined powder, and the mixture was subjected to thorough mixing. Then, the resultant mixture was carried and shaped in a spherical shape at an active mass ratio of 50 mass% on an inert carrier by using a 33 mass% glycerin solution as a binder by a tumbling granulation. The thus-obtained spherical shaped product having a particle size of 5.3 mm was subjected to main calcination under conditions of 550°C and 4 hours to obtain a catalyst 5.

[Reference Example 1]

In 380 parts by mass of pure water heated to 60°C, 100 parts by mass of ammonium heptamolybdate was completely dissolved (mother liquid 1). Next, 0.45 parts by mass of potassium nitrate was dissolved in 45 parts by mass of pure water, and the solution was added to the mother liquid 1. Next, 38 parts by mass of ferric nitrate, 71 parts by mass of cobalt nitrate, and 38 parts by mass of nickel nitrate were dissolved in 76 parts by mass of pure water heated to 60°C, and the solution was added to the mother liquid 1. Subsequently, 36 parts by mass of bismuth nitrate was dissolved in a nitric acid aqueous solution prepared by adding 9.7 parts by mass of nitric acid (60 mass%) to 41 parts by mass of pure water heated to 60°C, and the solution was added to the mother liquid 1. This mother liquid 1 was dried by a spray drying, and the obtained dry powder was subjected to preliminary calcination under conditions of 440°C and 4 hours. To the thus-obtained preliminarily calcined powder (atomic ratio calculated based on the charged raw materials: Mo:Bi:Fe:Co:Ni:K = 12:1.6:2.0:5.2:2.8:0.10), a crystalline cellulose was added in an amount of 5 mass% with respect to the preliminarily calcined powder, the mixture was subjected to thorough mixing. Then, the resultant mixture was carried and shaped in a spherical shape at an active mass ratio of 50 mass% on an inert carrier by using a 33 mass% glycerin solution as a binder by a tumbling granulation. The thus-obtained spherical shaped product having a particle size of 5.3 mm was subjected to main calcination under conditions of 530°C for 4 hours to obtain a catalyst 6.

[Comparative Example 2]

In 380 parts by mass of pure water heated to 60°C, 100 parts by mass of ammonium heptamolybdate was completely dissolved (mother liquid 1). Next, 0.17 parts by mass of potassium nitrate was dissolved in 1.9 parts by mass of pure water, and the solution was added to the mother liquid 1. Next, 89 parts by mass of cobalt nitrate and 33 parts by mass of nickel nitrate were dissolved in 73 parts by mass of pure water heated to 60°C, and the solution was added to the mother liquid 1. Next, 1.2 parts by mass of nitric acid (60 mass%) was added to the mother liquid 1 to adjust the pH to 3.0. Subsequently, 16 parts by mass of bismuth nitrate and 41 parts by mass of ferric nitrate were dissolved in a nitric acid aqueous solution prepared by adding 6.5 parts by mass of nitric acid (60 mass%) to 36 parts by mass of pure water heated to 60°C, and the solution was added to the mother liquid 1. This mother liquid 1 was dried by a spray drying, and the obtained dry powder was subjected to preliminary calcination under conditions of 440°C and 4 hours. To the thus-obtained preliminarily calcined powder (atomic ratio calculated based on the charged raw materials: Mo:Bi:Fe:Co:Ni:K = 12:0.7:2.2:6.5:2.4:0.04), a crystalline cellulose was added in an amount of 5 mass% with respect to the preliminarily calcined powder, and the mixture was subjected to thorough mixing. Then, the resultant mixture was carried and shaped in a spherical shape at an active mass ratio of 50 mass% on an inert carrier by using a 33 mass% glycerin solution as a binder by a tumbling granulation. The thus-obtained spherical shaped product having a particle size of 5.3 mm was subjected to main calcination under conditions of 530°C and 4 hours to obtain a catalyst 7.

[Comparative Example 3]

In 380 parts by mass of pure water heated to 60°C, 100 parts by mass of ammonium heptamolybdate was completely dissolved (mother liquid 1). Next, 0.17 parts by mass of potassium nitrate was dissolved in 1.9 parts by mass of pure water, and the solution was added to the mother liquid 1. Next, 100.0 parts by mass of 28% ammonia water was added to the mother liquid 1 to adjust the pH to 6.15. Next, 89 parts by mass of cobalt nitrate and 33 parts by mass of nickel nitrate were dissolved in 73 parts by mass of pure water heated to 60°C, and the solution was added to the mother liquid 1. Subsequently, 16 parts by mass of bismuth nitrate was dissolved in a nitric acid aqueous solution prepared by adding 4.1 parts by mass of nitric acid (60 mass%) to 17 parts by mass of pure water heated to 60°C, and the solution was added to the mother liquid 1. Further, 41 parts by mass of ferric nitrate was dissolved in 160 parts by mass of pure water heated to 60°C, and the solution was added to the mother liquid 1. This mother liquid 1 was dried by a spray drying, and the obtained dry powder was subjected to preliminary calcination under conditions of 440°C and 4 hours. To the thus-obtained preliminarily calcined powder (atomic ratio calculated based on the charged raw materials: Mo:Bi:Fe:Co:Ni:K = 12:0.7:2.2:6.5:2.4:0.04), a crystalline cellulose was added in an amount of 5 mass% with respect to the preliminarily calcined powder, and the mixture was subjected to thorough mixing. Then, the resultant mixture was carried and shaped in a spherical shape at an active mass ratio of 50 mass% on an inert carrier by using a 33 mass% glycerin solution as a binder by a tumbling granulation. The thus-obtained spherical shaped product having a particle size of 5.3 mm was subjected to main calcination under conditions of 530°C and 4 hours to obtain a catalyst 8.

Using the catalyst 1 to the catalyst 5, the catalyst 7, and the catalyst 8, the oxidation reaction of propylene was carried out by the following method, and the raw material conversion rate and the useful yield were determined. The gas inlet side of a stainless steel reaction tube having an inner diameter of 28 mm was filled with the catalyst 6 and the gas outlet side was filled with each of the catalyst 1 to the catalyst 5. And, a mixed gas containing propylene, oxygen, water vapor, and nitrogen in a gas volume ratio of 1.00:1.65:1.27:9.53 was introduced into the reaction tube at a propylene space velocity of 100 hr$^{-1}$ with respect to all the catalysts, to carry out the oxidation reaction of propylene. An aging reaction was performed at a salt bath temperature of 315°C for 20 hours or longer from the start of the reaction, and then, the gas at the outlet of the reaction tube was analyzed at a salt bath temperature of 320°C to obtain the raw material conversion rate and the useful yield shown in Table 1. Table 1 also shows the results of Raman spectroscopic measurement results. The Raman spectroscopic measurements on the catalyst 1 to the catalyst 5, the catalyst 7, and the catalyst 8 were carried out using the equipment and conditions exemplified above.

[Table 1]

|  | Catalyst | R1 | R2 | R3 | Propylene conversion rate/% | Useful yield/% | Useful selectivity/% |
|---|---|---|---|---|---|---|---|
| Example 1 | Catalyst 1 | 0.47 | 951.5 | 0.38 | 97.0 | 92.1 | 94.9 |
| Example 2 | Catalyst 2 | 0.47 | 951.4 | 0.35 | 97.0 | 91.9 | 94.7 |
| Example 3 | Catalyst 3 | 0.53 | 952.4 | 0.86 | 96.0 | 92.4 | 95.3 |
| Example 4 | Catalyst 4 | 0.50 | 951.9 | 0.60 | 96.7 | 91.9 | 94.8 |
| Comparative Example 1 | Catalyst 5 | 0.43 | 951.3 | 0.20 | 97.2 | 91.5 | 94.4 |
| Comparative Example 2 | Catalyst 7 | 0.44 | 951.1 | 0.16 | 97.4 | 91.3 | 94.1 |
| Comparative Example 3 | Catalyst 8 | 0.42 | 951.0 | 0.07 | 97.1 | 91.2 | 94.0 |

[Table 2]

| | Catalyst | p-0 | w-0 | i-1 | p-1 | w-1 | i-2 | p-2 | w-2 | i-3 | p-3 | w-3 | i-4 | p-4 | w-4 | i-5 |
|---|---|---|---|---|---|---|---|---|---|---|---|---|---|---|---|---|
| Example 1 | Catalyst 1 | 353.5 | 62.8 | 2.07 | 783.0 | 20.2 | 1.14 | 820.7 | 21.6 | 0.48 | 868.6 | 24.8 | 0.47 | 885.4 | 20.4 | 0.05 |
| Example 2 | Catalyst 2 | 353.4 | 61.8 | 2.08 | 783.0 | 20.1 | 1.14 | 820.7 | 21.6 | 0.48 | 868.6 | 24.7 | 0.47 | 885.4 | 20.4 | 0.05 |
| Example 3 | Catalyst 3 | 354.2 | 64.6 | 2.02 | 783.3 | 21.0 | 1.12 | 821.1 | 23.0 | 0.34 | 868.6 | 29.2 | 0.53 | 886.7 | 22.7 | 0.08 |
| Example 4 | Catalyst 4 | 354.4 | 66.2 | 1.57 | 782.9 | 21.9 | 1.08 | 820.5 | 24.3 | 0.39 | 868.1 | 32.8 | 0.50 | 885.4 | 22.5 | 0.10 |
| Comparative Example 1 | Catalyst 5 | 354.0 | 68.4 | 1.85 | 782.9 | 21.4 | 1.02 | 820.9 | 22.3 | 0.56 | 869.2 | 25.6 | 0.43 | 886.9 | 21.0 | 0.06 |
| Comparative Example 2 | Catalyst 7 | 352.9 | 62.3 | 2.12 | 782.8 | 20.7 | 1.05 | 820.4 | 22.2 | 0.47 | 868.4 | 23.8 | 0.44 | 885.0 | 20.1 | 0.08 |
| Comparative Example 3 | Catalyst 8 | 353.4 | 63.6 | 1.88 | 782.6 | 20.8 | 1.09 | 820.3 | 22.2 | 0.51 | 868.7 | 27.2 | 0.42 | 885.0 | 18.4 | 0.13 |

| | Catalyst | p-5 | w-5 | i-6 | p-6 | w-6 | i-7 | p-7 | w-7 | i-8 | p-8 | w-8 | i-9 | p-9 | w-9 |
|---|---|---|---|---|---|---|---|---|---|---|---|---|---|---|---|
| Example 1 | Catalyst 1 | 906.2 | 13.9 | 4.63 | 940.3 | 10.4 | 2.91 | 951.5 | 9.0 | 0.52 | 969.1 | 12.1 | 0.24 | 989.9 | 10.1 |
| Example 2 | Catalyst 2 | 906.2 | 13.9 | 4.65 | 940.3 | 10.4 | 2.92 | 951.4 | 9.0 | 0.52 | 969.1 | 12.1 | 0.24 | 990.0 | 10.2 |
| Example 3 | Catalyst 3 | 905.7 | 6.1 | 4.11 | 941.2 | 11.0 | 2.54 | 952.4 | 9.8 | 0.50 | 969.6 | 13.2 | 0.25 | 989.8 | 13.3 |
| Example 4 | Catalyst 4 | 903.2 | 16.7 | 4.24 | 940.7 | 11.8 | 2.57 | 951.9 | 10.3 | 0.53 | 968.8 | 15.0 | 0.20 | 987.8 | 8.9 |
| Comparative Example 1 | Catalyst 5 | 905.6 | 9.9 | 4.45 | 940.8 | 11.5 | 3.01 | 951.3 | 10.5 | 0.44 | 969.8 | 13.3 | 0.22 | 989.3 | 10.1 |
| Comparative Example 2 | Catalyst 7 | 902.5 | 20.6 | 4.36 | 940.1 | 11.0 | 2.76 | 951.1 | 9.5 | 0.55 | 968.5 | 12.8 | 0.26 | 989.4 | 10.6 |
| Comparative Example 3 | Catalyst 8 | 901.2 | 13.5 | 4.02 | 939.9 | 10.5 | 2.56 | 951.0 | 9.4 | 0.44 | 968.8 | 13.3 | 0.21 | 988.9 | 11.2 |

EP 4 302 873 A1

19

In the tables, the naming rules for each Raman parameter are as follows.

p-*: peak position of maximum value for giving *-th peak/cm$^{-1}$
w-*: peak width at *-th peak/cm$^{-1}$
i-*: (maximum value of peak at *-th peak) ÷ (maximum value of peak at 354 cm$^{-1}$ ± 5 cm$^{-1}$)

The assignment of the peaks marked with * in the above is as follows:

0: peak at 354 cm$^{-1}$ ± 5 cm$^{-1}$
1: peak at 782 cm$^{-1}$ ± 5 cm$^{-1}$
2: peak at 820 cm$^{-1}$ ± 5 cm$^{-1}$
3: peak at 868 cm$^{-1}$ ± 5 cm$^{-1}$
4: peak at 886 cm$^{-1}$ ± 5 cm$^{-1}$
5: peak at 900 cm$^{-1}$ ± 5 cm$^{-1}$
6: peak at 940 cm$^{-1}$ ± 4 cm$^{-1}$
7: peak at 952 cm$^{-1}$ ± 7 cm$^{-1}$
8: peak at 968 cm$^{-1}$ ± 5 cm$^{-1}$
9: peak at 989 cm$^{-1}$ ± 5 cm$^{-1}$
and R1 = i-4 and R2 = p-7.

It can be seen from Table 1 that the catalyst 1 to the catalyst 4, in which R1, R2, and R3 are within the ranges of the present invention, exhibit significantly higher useful yields. Further, the inventors have found that the relationship of the useful yield with respect to R1, R2, and R3 is represented by a generally linear relationship in which the useful yield increases as R1, R2, and R3 increases, and there are desirable ranges for these parameters in terms of useful yield.

Although the present invention has been described in detail with reference to specific embodiments, it will be apparent to those skilled in the art that various changes and modifications can be made without departing from the spirit and scope of the present invention.

This application is based on a Japanese patent application (Japanese Patent Application No. 2021-33249) filed on March 3, 2021, the entirety of which is incorporated by reference. In addition, all references cited herein are incorporated in entirety thereof.

INDUSTRIAL APPLICABILITY

Use of the present invention achieves a high yield, in the case of the use in a gas phase oxidation reaction, particularly in the case of the use in producing an unsaturated aldehyde compound or an unsaturated carboxylic acid compound by a partial oxidation reaction.

**Claims**

1. A catalyst comprising molybdenum, bismuth, and iron, wherein R1 represented by the following equation (1) is 0.45 or more and 5.00 or less,

$$\text{R1} = (\text{maximum value of peak at } 886 \text{ cm}^{-1} \pm 5 \text{ cm}^{-1}) \div (\text{maximum value of peak at } 354 \text{ cm}^{-1} \pm 5 \text{ cm}^{-1})$$

as measured by Raman spectroscopy        (1).

2. A catalyst comprising molybdenum, bismuth, and iron, wherein R2 represented by the following equation (2) is 951.4 cm$^{-1}$ or more and 959.0 cm$^{-1}$ or less,

$$\text{R2} = \text{wave number (cm}^{-1}) \text{ for giving maximum value of maximum peak at } 952 \text{ cm}^{-1} \pm 7 \text{ cm}^{-1} \text{ as}$$

measured by Raman spectroscopy        (2).

3. A catalyst comprising molybdenum, bismuth, and iron, wherein R3 represented by the following equation (3) is 0.21 or more,

R1 = (maximum value of peak at 886 cm$^{-1}$ ± 5 cm$^{-1}$) ÷ (maximum value of peak at 354 cm$^{-1}$ ± 5 cm$^{-1}$) as measured by Raman spectroscopy        (1),

R2 = wave number (cm$^{-1}$) for giving maximum value of maximum peak at 952 cm$^{-1}$ ± 7 cm$^{-1}$ as measured by Raman spectroscopy        (2),

$$R3 = 2.95 \times R1 + 0.33 \times R2 - 315 \qquad (3).$$

4.  The catalyst according to any one of claims 1 to 3, wherein a composition of a catalytically active component is represented by the following formula (I),

$$Mo_{a1}Bi_{b1}Ni_{c1}Co_{d1}Fe_{e1}X_{f1}Y_{g1}Z_{h1}O_{i1} \qquad (I)$$

where, Mo, Bi, Ni, Co, and Fe respectively represent molybdenum, bismuth, nickel, cobalt, and iron; X is at least one element selected from tungsten, antimony, tin, zinc, chromium, manganese, magnesium, silicon, aluminum, cerium, and titanium; Y is at least one element selected from sodium, potassium, cesium, rubidium, and thallium; Z belongs to Groups 1 to 16 in the periodic table, and means at least one element selected from elements other than the above Mo, Bi, Ni, Co, Fe, X, and Y; a1, b1, c1, d1, e1, f1, g1, h1, and i1 respectively represent the number of atoms of molybdenum, bismuth, nickel, cobalt, iron, X, Y, Z, and oxygen; and when a1 = 12, $0 < b1 \leq 7$, $0 \leq c1 \leq 10$, $0 < d1 \leq 10$, $0 < c1+d1 \leq 20$, $0 \leq e1 \leq 5$, $0 \leq f1 \leq 2$, $0 \leq g1 \leq 3$, $0 \leq h1 \leq 5$, and i1 is a value determined by an oxidation state of each element.

5.  The catalyst according to any one of claims 1 to 4, wherein the catalytically active component is carried on an inert carrier.

6.  The catalyst according to claim 5, wherein the inert carrier is silica, alumina, or a combination thereof.

7.  A method for producing the catalyst according to any one of claims 1 to 6, comprising:
    a blending step of adding each source compound containing molybdenum, bismuth and iron to a solvent or solution and performing integration and heating to form a blended solution, wherein, in the blending step, pH of the blended solution is adjusted to a range of 1.0 to 7.5 before adding an iron raw material.

8.  A method for producing an unsaturated aldehyde and/or an unsaturated carboxylic acid using the catalyst according to any one of claims 1 to 6.

**FIG. 1**

# FIG. 2

## INTERNATIONAL SEARCH REPORT

| International application No. |
|---|
| **PCT/JP2022/007535** |

**A. CLASSIFICATION OF SUBJECT MATTER**

*B01J 23/887*(2006.01)i; *B01J 37/04*(2006.01)i; *C07B 61/00*(2006.01)i; *C07C 45/35*(2006.01)i; *C07C 47/22*(2006.01)i; *C07C 51/25*(2006.01)i; *C07C 57/05*(2006.01)i
FI:   B01J23/887 Z; B01J37/04 102; C07C47/22 A; C07C45/35; C07C51/25; C07C57/05; C07B61/00 300

According to International Patent Classification (IPC) or to both national classification and IPC

**B. FIELDS SEARCHED**

Minimum documentation searched (classification system followed by classification symbols)
   B01J21/00-38/74; C07B61/00; C07C1/00-409/44

Documentation searched other than minimum documentation to the extent that such documents are included in the fields searched
   Published examined utility model applications of Japan 1922-1996
   Published unexamined utility model applications of Japan 1971-2022
   Registered utility model specifications of Japan 1996-2022
   Published registered utility model applications of Japan 1994-2022

Electronic data base consulted during the international search (name of data base and, where practicable, search terms used)
   JSTPlus/JST7580/ JSTChina (JDreamIII); CAplus (STN)

**C. DOCUMENTS CONSIDERED TO BE RELEVANT**

| Category* | Citation of document, with indication, where appropriate, of the relevant passages | Relevant to claim No. |
|---|---|---|
| X | SPRENGER, Paul, et al. Chemical Imaging of Mixed Metal Oxide Catalysts for Propylene Oxidation: From Model Binary Systems to Complex Multicomponent Systems. ChemCatChem. 01 March 2021, vol. 13, pp. 2483 -2493, DOI:10.1002/cctc.202100054 abstract, catalytic tests, spatially-resolved Raman spectroscopy and 2D mapping, fig. 4 | 1, 4, 8 |
| Y | | 5, 6 |
| A | | 2, 3, 7 |
| X | JP 2014-523809 A (BASF SE) 18 September 2014 (2014-09-18) claims, paragraphs [0056]-[0057], [0267]-[0290], fig. 5 | 1, 4-8 |
| Y | | 5, 6 |
| A | | 2, 3 |
| A | JP 2019-509889 A (EVONIK DEGUSSA GMBH) 11 April 2019 (2019-04-11) claims, example 5, fig. 7 | 1-8 |
| A | JP 2020-142221 A (MITSUBISHI CHEM CORP) 10 September 2020 (2020-09-10) claims, table 1 | 1-8 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **22 April 2022** | **10 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

EP 4 302 873 A1

INTERNATIONAL SEARCH REPORT
Information on patent family members

International application No.

**PCT/JP2022/007535**

| Patent document cited in search report | | | Publication date (day/month/year) | Patent family member(s) | | | Publication date (day/month/year) |
|---|---|---|---|---|---|---|---|
| JP | 2014-523809 | A | 18 September 2014 | US | 2013/0023699 | A1 | |
| | | | | claims, paragraphs [0076]-[0077], [0343]-[0380], fig. 5 | | | |
| | | | | EP | 2731715 | A1 | |
| | | | | CN | 103796752 | A | |
| | | | | KR | 10-2014-0057541 | A | |
| JP | 2019-509889 | A | 11 April 2019 | WO | 2017/157837 | A1 | |
| | | | | claims, example 5, fig. 7 | | | |
| | | | | US | 2019/0076829 | A1 | |
| | | | | EP | 3219386 | A1 | |
| | | | | CN | 109070061 | A | |
| JP | 2020-142221 | A | 10 September 2020 | (Family: none) | | | |

Form PCT/ISA/210 (patent family annex) (January 2015)

25

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016136882 A
- JP 2017024009 A
- WO 2015008815 A
- JP 2020142221 A
- JP 2021033249 A